(19) Europäisches Patentamt
European Patent Office
Office européen des brevets

(11) Veröffentlichungsnummer : **0 363 756 B1**

# (12) EUROPÄISCHE PATENTSCHRIFT

(45) Veröffentlichungstag der Patentschrift : **02.12.92 Patentblatt 92/49**

(51) Int. Cl.$^5$ : **C07C 229/62,** C07C 227/18

(21) Anmeldenummer : **89118109.1**

(22) Anmeldetag : **29.09.89**

## (54) Verfahren zur Herstellung von 2,5-Diarylaminoterephthalsäuren.

(30) Priorität : **12.10.88 DE 3834747**

(43) Veröffentlichungstag der Anmeldung : **18.04.90 Patentblatt 90/16**

(45) Bekanntmachung des Hinweises auf die Patenterteilung : **02.12.92 Patentblatt 92/49**

(84) Benannte Vertragsstaaten : **CH DE FR GB LI**

(56) Entgegenhaltungen :
**EP-A- 0 057 873**
**FR-A- 1 246 761**

(56) Entgegenhaltungen :
**FR-A- 1 328 683**
**GB-A- 975 466**
**CHEMICAL ABSTRACTS, Band 82, Nr. 25, 23. Juni 1975, Seiten 488-489, Zusammenfassung Nr. 170389u, Columbus, Ohio, US; &JP-A-74 108 036**

(73) Patentinhaber : **BAYER AG**
**W-5090 Leverkusen 1 Bayerwerk (DE)**

(72) Erfinder : **Schütze, Detlef-Ingo, Dr.**
**Roggendorfstrasse 51**
**W-5000 Koeln 80 (DE)**
Erfinder : **Schmitz, Reinold, Dr.**
**Im Kerberich 36**
**W-5068 Odenthal (DE)**

EP 0 363 756 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von 2,5-Diarylaminoterephthalsäuren, den wichtigen Zwischenprodukten zur Herstellung der wertvollen violetten oder roten Chinacridonpigmente.

Nach Liebermann (Liebigs Annalen, Band 404 (1914), S. 272) werden die 2,5-Diarylaminoterephthalsäuren durch Oxidation von 2,5-Diarylamino-3,6-dihydroterephthalsäuredialkylestern mit Jod in alkoholischer Lösung oder mit Luftsauerstoff in Eisessig/Alkohol und durch Verseifung der so erhaltenen und isolierten 2,5-Diarylaminoterephthalsäuredialkylester mit alkoholisch-wäßriger Lauge erhalten.

In der BE-PS 579 621 wurde dann ein vereinfachtes Verfahren beschrieben, wobei die 2,5-Diarylamino-3,6-dihydroterephthalsäuredialkylester in wäßrig-alkoholischer Lauge oxidiert und gleichzeitig zu den 2,5-Diarylaminoterephthalsäuren in einer einzigen Reaktionsstufe verseift werden. Als Oxidationsmittel wird dabei m-nitrobenzolsulfonsaures Natrium verwendet. Dabei ist mindestens 1 Mol des Oxidationsmittels pro Mol des eingesetzten 2,5-Diarylamino-3,6-dihydroterephthalsäureesters erforderlich. Außerdem wird erwähnt, daß an Stelle von m-nitrobenzolsulfonsaurem Natrium auch Luftsauerstoff als Oxidationsmittel verwendet werden kann. Das Verfahren ist dann jedoch viel weniger wirksam und bedeutend schwieriger zu kontrollieren (s. BE-PS 579 621, S. 7, Abs, 2, Zeile 8 bis 11). Auch weitere aromatische Nitroverbindungen wie beispielsweise m-Dinitrobenzol, p-Nitrotoluol, p-Nitrochlorbenzol u.a. werden als Oxidationsmittel in wäßrig-alkoholischen Laugen beschrieben (US-PS 3 388 149).

Um die Verwendung von aromatischen Nitroverbindungen vermeiden zu können, wurden weitere Versuche unternommen, Verfahren für die Oxidation mit Luft zu entwickeln. In der DE-AS 1 118 215 wird die Oxidation der 2,5-Diarylamino-3,6-dihydroterephthalsäuredialkylester direkt nach ihrer Bildung aus Succinylobernsteinsäuredialkylestern und dem jeweiligen Arylamin im überschüssigen Arylamin mit Luft vorgenommen. In einem zusätzlichen Schritt muß der entstandene und isolierte 2,5-Diarylaminoterephthalsäuredialkylester zur 2,5-Diarylaminoterephthalsäure verseift werden. Dieses Verfahren ist sehr aufwendig.

In der DE-AS 1 114 285 wurde dann ein Verfahren veröffentlicht, bei dem die Oxidation der 2,5-Diarylamino-3,6-dihydroterephthalsäuredialkylester in wäßrig-alkoholischen Laugen unter Zusatz von geringen Mengen Chinonsulfonsäuren, wie Naphthochinon-, Anthrachinon- und Phenanthrenchinonsulfonsäuren, als Sauerstoffüberträger mit Luft vorgenommen wird. Eine Erweiterung erfährt dieses Verfahren durch die DE-AS 1 147 953, indem als Sauerstoffüberträger direkt Chinone wie Anthrachinon, Phenanthrenchinon, Naphthochinon, Chloranil oder Chinoncarbonsäuren Verwendung finden. Gegenüber den übrigen Verfahren stellt dieses Luftoxidations-Verfahren zwar eine deutliche Verbesserung dar, es kann jedoch insbesondere für die Herstellung der substituierten 2,5-Dianilinoterephthalsäuren wie beispielsweise Di-4-toluidino- oder Di-4-chloranilinoterephthalsäuren nicht befriedigen. Zur Darstellung dieser beiden substituierten, 2,5-Diarylaminoterephthalsäuren wird als Lösungsmittel n-Butanol/Wasser eingesetzt, da die Oxidation in Methanol/Wasser oder Ethanol/Wasser mit Luft nicht vollständig durchgeführt werden kann. In n-Butanol/Wasser entsteht jedoch ein mehrphasiges System, durch das, beispielsweise bei der Nacharbeitung des Beispiels 2 der DE-AS 1 144 285, ein sehr schmieriges und verklumptes Produkt entsteht. Außerdem wird bedingt durch dieses mehrphasige System eine erheblich längere Oxidationszeit benötigt, als angegeben worden ist. Die Ausbeuten in beiden Auslegeschriften schwanken je nach Derivat zwischen 86 % und 93 %.

Überraschenderweise führt das erfindungsgemäße Verfahren mit hohen Ausbeuten zu 2,5-Diarylaminoterephthalsäuren hoher Reinheit bei deutlich kürzeren Oxidationszeiten. Das erfindungsgemäße Verfahren zur Herstellung von 2,5-Diarylaminoterephthalsäuren durch Oxidation von 2,5-Diarylamino-3,6-dihydroterephthalsäureestern mit Sauerstoff oder sauerstoffhaltigen Gasen, vorzugsweise Luft, in alkoholisch-alkalischer oder alkoholisch-wäßrig-alkalischer Lösung oder Suspension in Gegenwart eines sauerstoffübertragenden Mittels und Aufarbeitung zu den 2,5-Diarylaminoterephthalsäuren ist dadurch gekennzeichnet, daß man in Gegenwart einer quartären Ammoniumverbindung oxidiert. Eine Ausgestaltung dieses Verfahrens besteht darin, daß man in die Oxidation das 2,5-Diarylamino-3,6-dihydroterephthalsäureester-Bildungsgemisch, wie es bei der Umsetzung von 1 Mol Succinylobernsteinsäureester mit 2 Mol eines Arylamins, vorzugsweise in alkoholischer Lösung, erhalten wird, einsetzt.

Die Oxidationen werden vorzugsweise bei 70 bis 130°C, besonders bevorzugt beim Siedepunkt des Reaktionsgemischs, durchgeführt. Gegebenenfalls kann unter Druck gearbeitet werden.

Insbesondere dient das neue Verfahren zur Herstellung von 2,5-Diarylaminoterephthalsäuren der Formel

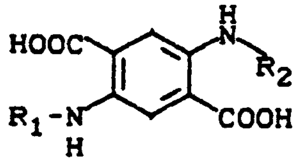

(I)

in der

$R_1 = R_2 =$ 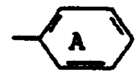

oder

$R_1 =$ 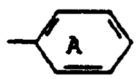 und $R_2 =$ 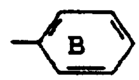

sind,
wobei die Ringe A und B durch 1 bis 4 Substituenten aus der Reihe $C_1$-$C_4$-Alkyl, Chlor, Fluor, $C_1$-$C_4$-Alkoxy, gegebenenfalls durch $C_1$-$C_4$-Alkyl mono- oder disubstituiertes Carbamoyl, Trifluormethyl, Carboxy, Nitro substituiert sein können oder an sie ein aromatischer oder heteroaromatischer Ring ankondensiert sein kann, wobei man vorzugsweise 2,5-Diarylamino-3,6-dihydroterephthalsäureester der Formel

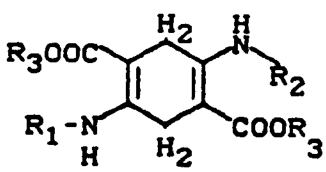

(II)

in der $R_1$, $R_2$ die oben angegebenen Bedeutungen haben und $R_3$ für Alkyl, vorzugsweise $C_1$-$C_6$-Alkyl, z.B. Methyl, Ethyl, i-Propyl, steht oder entsprechende Bildungsgemische in die Oxidation einsetzt.

Das erfindungsgemäße Verfahren läßt sich z.B. wie folgt ausführen: Der 2,5-Diarylamino-3,6-dihydroterephthalsäureester wird in einem Gemisch, bestehend aus einem Alkohol und einem Alkalihydroxid oder bestehend aus einem Alkohol, Wasser und einem Alkalihydroxid suspendiert, ein Sauerstoffüberträger, beispielsweise Anthrachinonsulfonsäure und zusätzlich eine quartäre Ammoniumverbindung hingezufügt, das Gemisch erhitzt und durch Durchleiten von Sauerstoff oder sauerstoffhaltigen Gasen wie beispielsweise Luft, gegebenenfalls unter Druck, oxidiert.

Eine weitere Ausführung des neuen Verfahrens besteht darin, daß man zu dem Bildungsgemisch zur Herstellung der 2,5-Diarylamino-3,6-dihydroterephthalsäureester, das aus Succinylobernsteinsäureester, Arylamin, Alkohol und beispielsweise etwas Eisessig zur Kondensation besteht, ein Alkalihydroxid oder eine Lösung eines Alkalihydroxids in Wasser, einem Sauerstoffüberträger, beispielsweise Anthrachinonsulfonsäure und eine quartäre Ammoniumverbindung hinzufügt, das Gemisch erhitzt und durch Durchleiten von Sauerstoff oder sauerstoffhaltigen Gasen wie beispielsweise Luft, gegebenenfalls unter Druck, oxidiert.

Als Arylamine werden bevorzugt Anilin, p-Toluidin, p-Chloranilin, p-Anisidin oder p-Phenetidin eingesetzt. Als Alkalihydroxide sind Natriumhydroxid und Kaliumhydroxid besonders bevorzugt.

Als Alkohole kommen vorzugsweise Methanol, Ethanol, Propanol, i-Propanol, n-Butanol, Glykole oder Glykolether, z.B. Ethylenglykol, Ethylenglykolmonomethylether und Polyglykole sowie Gemische dieser Alkohole in Betracht.

Als sauerstoffübertragende Mittel werden vorzugsweise eingesetzt: Chinone wie Anthrachinon, Phenanthrenchinon, Naphthochinon und Chloranil sowie deren Sulfon- und Carbonsäuren, die auch als Salze Verwendung finden können. Besonders bevorzugt ist die Verwendung von Anthrachinonmono- und disulfonsäuren, bzw. deren Salzen; ganz besonders bevorzugt wird Anthrachinon-2-sulfonsäure(salz) eingesetzt. Bezogen auf

2,5-Diarylamino-3,6-dihydroterephthalsäureester werden vorzugsweise 0,5 bis 5 Gew.-% sauerstoffübertragendes Mittel zugesetzt.

Als quartäre Ammoniumverbindungen werden vorzugsweise Verbindungen der Formeln

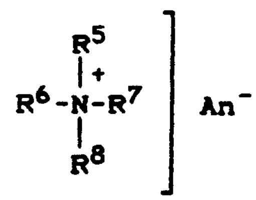 (III)

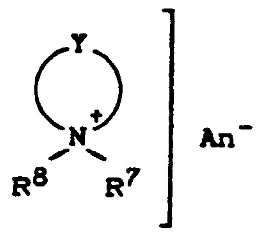 (IV)

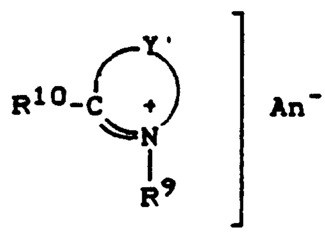 (V)

eingesetzt, wobei

$R^5$, $R^6$, $R^7$, $R^8$, $R^9$ $C_1$-$C_{54}$-Kohlenwasserstoffreste bezeichnen, deren C-Kette durch 1 bis 15 O-Atom(e) unterbrochen sein kann,

$R^{10}$ Wasserstoff oder einen Substituenten, z.B. $C_1$-$C_4$-Alkyl (Methyl, Ethyl) und

Y, Y′ die restlichen Glieder eines vorzugsweise 5- oder 6-gliedrigen Ringes, z.B. eines Pyridin- oder Imidazol-Ringes, oder eines Ringsystems und

$An^-$ einen anionischen Rest bezeichnen.

Als Reste $R^5$-$R^9$ seien insbesondere genannt : $C_1$-$C_{18}$-Alkyl, z.B. Methyl, Ethyl, Dodecyl, Cetyl, Phenyl, Phenyl-$C_1$-$C_{18}$-alkyl, z.B. Benzyl, $-CH_2CH_2O-(-CH_2-CH_2O-)_n-H$ mit n = 0 bis 20.

Als anionische Reste $An^-$ seien beispielhaft genannt : Chlorid, Sulfat, Methylsulfat, Benzolsulfonat, Toluolsulfonat, Hydroxid.

Im einzelnen sind zu nennen: Trimethylphenylammoniumchlorid, Triethylphenylammoniumchlorid, Triethylbenzylammoniumchlorid, Trimethylbenzylammoniumchlorid, Dimethyldibenzylammoniumchlorid, Diethyldibenzylammoniumchlorid, Dodecyltrimethylammoniumchlorid, Dodecyldimethylbenzylammoniumchlorid, mit Dimethyl- oder Diethylsulfat quarternierte Anlagerungsprodukte von 5 bis 10 Mol Ethylenoxid an $C_{16}$-$C_{20}$-Alkylamine, Dodecylbenzylalkylamine und Dodecylbenzylamin, Benzylpyridiniumchlorid, Dodecylpyridiniumchlorid und Cetylpyridiniumchlorid.

Auch die Hydrogensulfate, Sulfate, Methylsulfate, Ethylsulfate, Benzol- oder Toluolsulfonate und Hydroxide können statt der genannten Chloride mit gleichem Erfolg eingesetzt werden.

Naturgemäß kann das erfindungsgemäße Verfahren auch in Gegenwart von Mischungen quartärer Ammoniumverbindungen und/oder Mischungen von sauerstoffübertragenden Mitteln durchgeführt werden.

Die Mengen an quartären Ammoniumsalzen liegen - bezogen auf den zu oxidierenden Ester - im allgemeinen zwischen 0,1 und 15, vorzugsweise zwischen 0,5 und 8 Gew.-%.

Kationische Reste der Formeln (III) bis (V) sind z.B.: Tri-$C_1$-$C_4$-alkyl-phenylammonium; Di-$C_1$-$C_4$-alkyl-dibenzylammonium, $C_{10}$-$C_{16}$-Alkyl-tri-$C_1$-$C_4$-alkylammonium, Trialkylammoniumpolyglykolether, Dialkylbenzylammoniumpolyglykolether, Alkylbenzylammoniumdipolyglykolether, N-$C_{10}$-$C_{15}$-Alkylpyridinium, N-Phenyl-$C_7$-$C_{10}$-alkylpyridinium sowie kationische Reste von Quaternierungsprodukten von $C_1$-$C_4$-Trialkylammonium, N-$C_1$-$C_{18}$-Alkylimidazolium. Die genannten kationischen Reste können z.B. in Form der Chloride, Sulfate, Methylsulfate, Toluolsulfonate, Benzolsulfonate oder Hydroxide vorliegen. Gleichzeitig mit der Oxidation erfolgt in den meisten Fällen die Verseifung zur 2,5-Diarylaminoterephthalsäure. Anderfalls kann auf die übliche Weise verseift werden. Es entsteht in vielen Fällen eine klare Lösung. Das Ende der Reaktion läßt sich chromatografisch bestimmen.

Bezogen auf 1 Gew.-Teil des eingesetzten 2,5-Diarylamino-3,6-dihydroterephthalsäuredialkylesters werden bevorzugt 1 - 20 Gew.-Teile, besonders bevorzugt 4 bis 12 Gew.-Teile Lösungsmittel und bevorzugt 0,1 bis 2,5 Gew.-Teile, besonders bevorzugt 0,3 bis 1,5 Gew.-Teile Alkali, z.B. NaOH oder KOH, eingesetzt.

Die Aufarbeitung des Ansatzes kann z.B. wie folgt durchgeführt werden: Aus der vorliegenden Lösung der Dialkalisalze werden die 2,5-Diarylaminoterephthalsäuren durch einen Überschuß an Säure, wie beispielsweise Salzsäure oder Schwefelsäure, gegebenenfalls nach Verdünnung mit Wasser und Klärfiltration, freigesetzt und durch Filtration isoliert.

Das erfindungsgemäße Verfahren liefert die 2,5-Diarylaminoterephthalsäuren in sehr guten Ausbeuten und in sehr hoher Reinheit bereits nach sehr kurzen Reaktionszeiten. Sie können ohne eine weitere Reinigung, z.B. nach den in der US-PS 3 342 823 genannten Verfahren, direkt zu Chinacridon-Pigmenten umgesetzt werden.

Das erfindungsgemäße Verfahren wird durch die folgenden Ausführungsbeispiele weiter erläutert. Die darin angegebenen Teile und Prozentangaben beziehen sich auf das Gewicht.

## Beispiel 1

In 115 Teilen Methanol werden 30 Teile 2,5-Dianilino-3,6-dihydroterephthalsäurediethylester, 93 Teile 14%ige Natronlauge und 1,5 Teile Anthrachinon-2-sulfonsäure eingetragen. Nach Zusatz von 4 Teilen einer 50%igen Lösung von Dodecylbenzyldimethylammoniumchlorid in Wasser wird die Suspension zum Sieden erhitzt und 10 l Luft/Stunde durchgeleitet. Nach 30 Minuten ist die Reaktion beendet und Lösung eingetreten.

Zur Isolierung des Produktes werden zunächst 90 Teile Methanol abdestilliert und die verbleibende Reaktionslösung bei Raumtemperatur mit 66 Teilen 25%iger Schwefelsäure kongosauer gestellt. Das ausgefällte Produkt wird abgesaugt, mit Wasser neutral gewaschen und getrocknet.

Man erhält 25,5 Teile (= 99 % der Theorie) der 2,5-Dianilinoterephthalsäure als violettes, kristallines Pulver.

Wird dieses Beispiel wiederholt ohne Zusatz des quartären Ammoniumsalzes, so werden 3 Stunden zur Oxidation benötigt.

## Beispiel 2

395 Teile Methanol, 36 Teile Succinylobernsteinsäuredimethylester, 39 Teile Anilin und 15,3 Teile Eisessig werden in einem Rührwerksautoklaven 4 Stunden auf 102 bis 105°C erhitzt.

Es wird abgekühlt, 42,5 Teile Kaliumhydroxid gelöst in 79 Teilen Methanol, 3 Teile Anthrachinon-2-sulfonsäure und 5 Teile einer 50%igen Lösung von Dodecylbenzyldimethylammoniumchlorid zugesetzt und zum Sieden erhitzt. Nachdem 3 Stunden unter Durchleitung von 10 l Luft/Stunde oxidiert worden ist, wird mit 350 Teilen Wasser verdünnt, die Lösung geklärt und mit 95 Teilen konzentrierter Salzsäure kongosauer gestellt. Die ausgefallene 2,5-Dianilinoterephthalsäure wird abgesaugt, mit Wasser neutral gewaschen und getrocknet.

Man erhält 53,9 Teile (= 98 % der Theorie) and 2,5-Dianilinoterephthalsäure.

Wird der gleiche Versuch ohne Zusatz des quartären Ammoniumsalzes durchgeführt, so werden nach 6 Stunden 48,9 Teile (= 89 % der Theorie) erhalten.

## Beispiel 3

In 152 Teile n-Butanol werden 29,9 Teile 2,5-Di-4-toluidino-3,6-dihydroterephthalsäuredimethylester, 114 Teile 22%ige Natronlauge und 1,5 Teile Anthrachinon-1,5-disulfonsäure eingetragen. Nach Zusatz von 4 Teilen einer 50%igen Lösung von Dodecylbenzyldimethylammoniumchlorid in Wasser wird auf 80 bis 90°C erhitzt und 7 Stunden unter Durchleiten von 10 l Luft/Stunde oxidiert. Anschließend werden etwa 220 Teile eines azeotropen Gemisches von n-Butanol und Wasser abdestilliert, wobei gleichzeitig etwa 220 Teile Wasser zugefügt werden. Danach wird mit 66 Teilen 25%iger Schwefelsäure kongoneutral gestellt, abgesaugt, mit Wasser neutral gewaschen und getrocknet. Man erhält 27 Teile (= 97,5 % der Theorie) an 2,5-Di-4-toluidinoterephthalsäure als violettes Pulver. Wird dieses Beispiel ohne Zusatz des quartären Ammoniumsalzes durchgeführt, so wird eine Ausbeute von 19,8 Teilen (= 71,5 % der Theorie) nach Oxidation erhalten.

Beispiel 4

410 Teile Ethanol, 52,5 Teile p-Toluidin, 41,9 Teile Succinylobernsteinsäuredimethylester und 17,7 Teile Eisessig werden in einem Rührwerksautoklaven 4 Stunden auf 102 bis 105°C erwärmt. Anschließend werden nach dem Abkühlen 66 Teile Waser, 49 Teile Kaliumhydroxid, 3 Teile Anthrachinon-2-sulfonsäure und 2,5 Teile Dipolyglykoldodecylbenzylammoniumchlorid (Soluofen-VV-308 der GAF Corporation) zugesetzt und 3 Stunden unter Rückfluß mit 10 l Luft/Stunde oxidiert. Danach werden 300 Teile Wasser zugefügt, die Lösung geklärt und das Produkt mit 89 Teilen konzentrierter Salzsäure gefällt. Nach dem Absaugen, Neutralwaschen und Trocknen werden 66,4 Teile (= 96,1 % der Theorie) an 2,5-Di-4-toluidinoterephthalsäure erhalten.

Beispiel 5

In 315 Teile Ethanol werden 36 Teile Succinylobernsteinsäuredimethylester, 45 Teile p-Toluidin und 15 Teile Eisessig eingetragen. Das Gemisch wird in einem Rührwerksautoklaven 4 Stunden bei 102 bis 105°C gerührt. Nach dem Abkühlen werden 200 Teile Ethanol, 60 Teile Wasser, 42,5 Teile Kaliumhydroxid, 3 Teile Anthrachinon-2-sulfonsäure und 4 g einer 50%igen Lösung von Dodecylbenzyldimethyldiammoniumchlorid in Wasser zugefügt. Unter Rückfluß wird 2,5 Stunden unter Durchleiten von 10 l Luft/Stunde oxidiert. Anschließend wird mit 300 Teilen Wasser verdünnt, geklärt und das Produkt mit 83 Teilen konzentrierter Salzsäure ausgefällt. Nach dem Absaugen, Neutralwaschen und Trocknen werden 58,5 Teile (= 98,5 % der Theorie) an 2,5-Di-4-toluidinoterephthalsäure erhalten.

Beispiel 6

408 Teile Isopropanol, 52,5 Teile p-Toluidin, 41,9 Teile Succinylobernsteinsäuredimethylester und 18 Teile Eisessig werden im Rührwerksautoklaven 4 Stunden auf 102 bis 105°C erwärmt. Nach dem Abkühlen werden 66 Teile Wasser, 49 Teile Kaliumhydroxid, 3 Teile Anthrachinon-2-sulfonsäure und 2,5 Teile Benzyltriethylammoniumchlorid hinzugegeben. Unter Rückfluß wird 3 Stunden mit 10 l Luft/Stunde oxidiert. Nach Zugabe von 600 Teilen Wasser und Klärung wird mit 89 Teilen konzentrierter Salzsäure kongoneutral gestellt. Das ausgefallene Produkt wird abgesaugt, mit Wasser neutral gewaschen und getrocknet. Man erhält 65,9 Teile (= 95,4 % der Theorie) an 2,5-Di-4-toluidinoterephthalsäure.

Beispiel 7

Wird das Beispiel 6 wiederholt und statt 2,5 g Benzyltriethylammoniumchlorid 2,5 g Benzyltrimethylammoniumhydroxid eingesetzt, so werden nach ebenfalls 3 Stunden Oxidationszeit 66,1 Teile (= 95,7 % der Theorie) an 2,5-Di-4-toluidinoterephthalsäure erhalten.

Beispiel 8

In 430 Teilen Methanol werden 95 Teile p-Chloranilin, 72,4 Teile Succinylobernsteinsäuredimethylester und 26 Teile Eisessig verrührt und in einem Rührwerksautoklaven 4 Stunden auf 90 bis 95°C erhitzt. Nach dem Abkühlen werden 130 Teile Methanol, 183 Teile 45%ige Kalilauge, 1 Teil Anthrachinon-2-sulfonsäure und 10 Teile einer 50%igen Lösung von Dodecylbenzyldimethylammoniumchlorid in Wasser zugesetzt. Unter Rückflußtemperatur wird mit 10 l Luft/Stunde 5 Stunden oxidiert. Anschließend werden 650 Teile Wasser zugesetzt, die Lösung geklärt und mit 160 Teilen konzentrierter Salzsäure kongoneutral gestellt. Nach dem Absaugen, Neutralwaschen und Trocknen erhält man 126,7 Teile (= 95,7 % der Theorie) der roten, kristallinen 2,5-Di-4-chloranilino-terephthalsäure. Wird dieses Beispiel ohne Zusatz des quartären Ammoniumsalzes wiederholt, so erhält man nach 11 Stunden 124,2 Teile (= 93,8 % der Theorie) an 2,5-Di-4-chloranilinoterephthalsäure.

Beispiel 9

620 Teile Ethanol, 102 Teile p-Anisidin, 81 Teile Succinylobernsteinsäuredimethylester und 30 Teile Eisessig werden in einem Rührwerksautoklaven 5 Stunden auf 100 bis 103°C geheizt. Nach dem Abkühlen werden 94 Teile Kaliumhydroxid, 125 Teile Wasser, 3 Teile Anthrachinon-2-sulfonsäure und 5 Teile Dipolyglykoldodecylbenzylammoniumchlorid (Soluofen-VV-308 der GAF Corporation) zugesetzt und 3 Stunden unter Rückfluß mit 10 ltr. Luft/Stunde oxidiert. Anschließend wird mit 600 Teilen Wasser verdünnt, die Lösung geklärt und mit 155 Teilen konzentrierter Salzsäure kongosauer gestellt. Das ausgefallene Produkt wird abgesaugt, neutral gewaschen und getrocknet. Man erhält 142 Teile (= 98 % der Theorie) der violetten, kristallinen, 2,5-Di-4-anisidinoterephthalsäure. Wird dieses Beispiel ohne den Zusatz des quartären Ammoniumsalzes durchgeführt, so werden nach 10 Stunden Oxidationszeit 135 Teile (= 93,2 % der Theorie) an 2,5-Di-4-anisidinoterephthalsäure erhalten.

## Patentansprüche

1. Verfahren zur Herstellung von 2,5-Diarylaminoterephthalsäuren durch Oxidation von 2,5-Diarylamino-3,6-dihydroterephthalsäuredialkylestern mit Sauerstoff oder sauerstoffhaltigen Gasen, vorzugsweise Luft, in alkoholisch-alkalischer oder alkoholisch-wäßrig-alkalischer Lösung oder Suspension in Gegenwart eines sauerstoffübertragenden Mittels und Aufarbeitung zu den 2,5-Diarylaminoterephthalsäuren, dadurch gekennzeichnet, daß man in Gegenwart einer quartären Ammoniumverbindung oxidiert.

2. Verfahren gemäß Anspruch 1 zur Herstellung von 2,5-Diarylaminoterephthalsäuren der Formel (I)

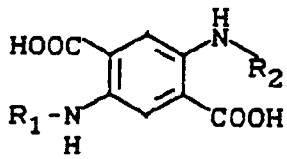

(I)

in der

$R_1$ = $R_2$ = 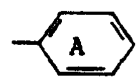

oder

$R_1$ = 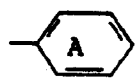 und $R_2$ = 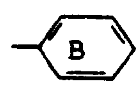

sind, wobei die Ringe A und B durch 1 bis 4 Substituenten aus der Reihe $C_1$-$C_4$-Alkyl, Chlor, Fluor, $C_1$-$C_4$-Alkoxy, gegebenenfalls durch $C_1$-$C_4$-Alkyl mono- oder disubstituiertes Carbamoyl, Trifluormethyl, Carboxyl, Nitro substituiert sein können oder an sie ein aromatischer oder heteroaromatischer Ring ankondensiert sein kann, durch Oxidation von 2,5-Diarylamino-3,6-dihydroterephthalsäureestern der Formel

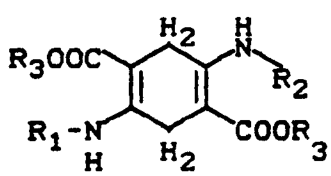

(II)

in der $R_1$, $R_2$ die oben angegebenen Bedeutungen haben und $R_3$ für Alkyl steht.

3. Verfahren gemäß den Ansprüchen 1 bis 2, dadurch gekennzeichnet, daß man in die Oxidation das 2,5-Diarylamino-3,6-dihydroterephthalsäureester-Bildungsgemisch, wie es bei der Umsetzung von Succinylobernsteinsäureester mit einem Arylamin erhalten wird, einsetzt.

4. Verfahren gemäß einem oder mehreren der Ansprüche 1 bis 3, dadurch gekennzeichnet, daß man eine quartäre Ammoniumverbindung der Formeln

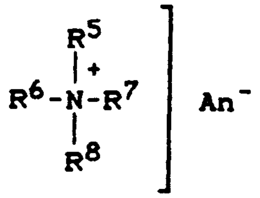

(III)

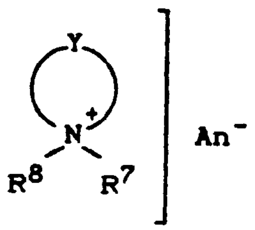

(IV)

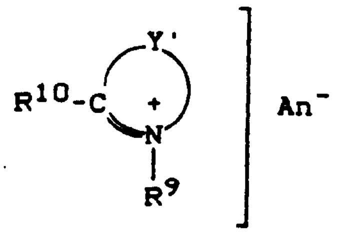

(V)

einsetzt, wobei

$R^5$, $R^6$, $R^7$, $R^8$, $R^9$ $C_1$-$C_{54}$-Kohlenwasserstoffreste bezeichnen, deren C-Kette durch 1 bis 15 O-Atom(e) unterbrochen sein kann,

$R^{10}$ Wasserstoff oder einen Substituenten,

Y, Y′ die restlichen Glieder eines vorzugsweise 5-oder 6-gliedrigen Ringes oder eines Ringsystems und

$An^-$ einen anionischen Rest bezeichnen.

5. Verfahren gemäß einem oder mehreren der Ansprüche 1 bis 4, dadurch gekennzeichnet, daß man, bezogen auf 2,5-Diarylamino-3,6-dihydroterephthalsäureester, 0,1 bis 15 Gew.-%, vorzugsweise 0,5 bis 8 Gew.-% quartäre Ammoniumverbindung einsetzt.

6. Verfahren gemäß einem oder mehreren der Ansprüche 1 bis 5, dadurch gekennzeichnet, daß man als sauerstoffübertragendes Mittel Anthrachinon-2-sulfonsäure einsetzt.

7. Verfahren gemäß einem oder mehreren der Ansprüche 1 bis 6, dadurch gekennzeichnet, daß man als Alkohol Methanol, Ethanol oder i-Propanol einsetzt.

8. Verfahren gemäß einem oder mehreren der Ansprüche 1 bis 7, dadurch gekennzeichnet, daß man bei 70 bis 130°C oxidiert.

9. Verfahren gemäß einem oder mehreren der Ansprüche 1 bis 8, dadurch gekennzeichnet, daß man beim Siedepunkt des Reaktionsgemisches oxidiert.

## Claims

1. Process for the preparation of 2,5-diarylaminoterephthalic acids by oxidation of 2,5-diarylamino-3,6-dihydroterephthalic acid dialkyl esters with oxygen or oxygen-containing gases, preferably air, in alcoholic-alkaline or alcoholic-aqueous-alkaline solution or suspension in the presence of an oxygen- transferring agent and working up to give the 2,5- diarylaminoterephthalic acids, characterized in that the oxidation is carried out in the presence of a quaternary ammonium compound.

2. Process according to Claim 1 for the preparation of 2,5-diarylaminoterephthalic acids of the formula (I)

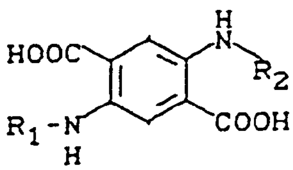

(I)

in which

$R_1 = R_2 =$ 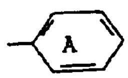

or

$R_1 =$ 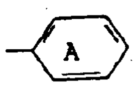 and $R_2 =$ 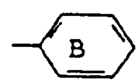

where the rings A and B may be substituted by 1 to 4 substituents from the series comprising $C_1$-$C_4$-alkyl, chlorine, fluorine, $C_1$-$C_4$-alkoxy, carbamoyl which is optionally monosubstituted or disubstituted by $C_1$-$C_4$-alkyl, trifluoromethyl, carboxyl or nitro, or may be fused to an aromatic or heteroaromatic ring, by oxidation of 2,5-diarylamino-3,6-dihydroterephthalic acid esters of the formula

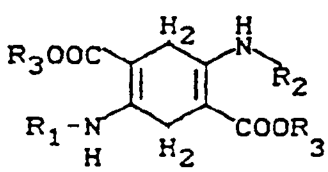

(II)

in which $R_1$ and $R_2$ have the abovementioned meanings and $R_3$ represents alkyl.

3. Process according to Claims 1 to 2, characterized in that the 2,5-diarylamino-3,6-dihydroterephthalic acid ester formation mixture is employed in the oxidation as it is obtained in the reaction of succinylosuccinic acid esters with an arylamine.

4. Process according to one or more of Claims 1 to 3, characterized in that a quaternary ammonium compound of the formulae

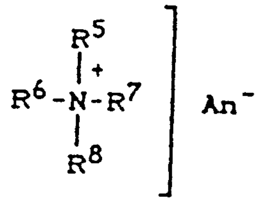

(III)

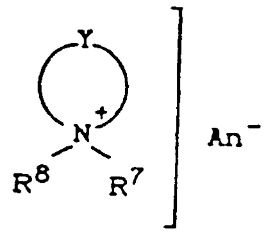

(IV)

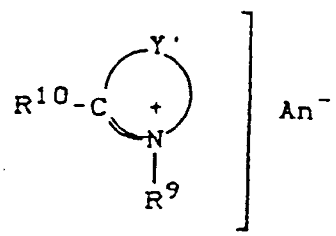

(V)

is employed in which

$R^5$, $R^6$, $R^7$, $R^8$ and $R^9$ signify $C_1$-$C_{54}$ hydrocarbon radicals, the C chain of which may be interrupted by 1 to 15 O atom(s),

$R^{10}$ signifies hydrogen or a substituent,

Y and Y′ signify the remaining member of a preferably 5-or 6-membered ring or a ring system and

$An^-$ signifies an anionic radical.

**5.** Process according to one or more of Claims 1 to 4, characterized in that 0.1 to 15 % by weight, preferably 0.5 to 8 % by weight, of quaternary ammonium compound are employed, relative to 2,5- diarylamino-3,6-dihydroterephthalic acid ester.

**6.** Process according to one or more of Claims 1 to 5, characterized in that anthraquinone-2-sulphonic acid is employed as the oxygen-transferring agent.

**7.** Process according to one or more of Claims 1 to 6, characterized in that methanol, ethanol or i-propanol is employed as the alcohol.

**8.** Process according to one or more Claims 1 to 7, characterized in that the oxidation is carried out at 70 to 130°C.

**9.** Process according to one or more of Claims 1 to 8, characterized in that the oxidation is carried out at the boiling point of the reaction mixture.

## Revendications

**1.** Procédé de préparation d'acides 2,5-diarylamino-téréphtaliques par oxydation d'esters dialkyliques d'acides 2,5-diarylamino-3,6-dihydrotéréphtaliques avec de l'oxygène ou des gaz contenant de l'oxygène, de préférence de l'air, en solution ou suspension alcoolique-alcaline ou alcoolique-aqueuse-alcaline en présence d'un agent de transfert d'oxygène et traitement final pour obtenir les acides 2,5-diarylaminotéréphtaliques, caractérisé en ce qu'on effectue l'oxydation en présence d'un composé d'ammonium quaternaire.

2. Procédé suivant la revendication 1 pour la préparation d'acides 2,5-diarylaminotéréphtaliques de formule (I)

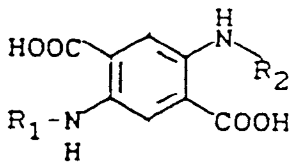

(I)

dans laquelle

$R_1 = R_2 =$ 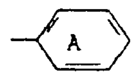

ou

$R_1 =$ 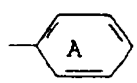 et $R_2 =$ 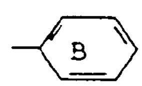 ,

les noyaux A et B pouvant porter 1 à 4 substituants de la série alkyle en $C_1$ à $C_4$, chloro, fluoro, alkoxy en $C_1$ à $C_4$, carbamoyle portant éventuellement un ou deux substituants alkyle en $C_1$ à $C_4$, trifluorométhyle, carboxyle, nitro, ou pouvant être liés par condensation à un noyau aromatique ou hètéro-aromatique, par oxydation d'esters d'acides 2,5-diarylamino-3,6-dihydrotéréphtaliques de formule

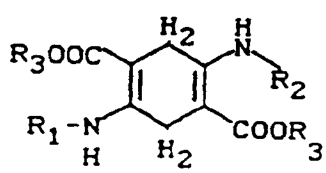

(II)

dans laquelle $R_1$, $R_2$ ont les définitions indiquées ci-dessus et $R_3$ est un groupe alkyle.

3. Procédé suivant les revendications 1 et 2, caractérisé en ce qu'on utilise dans l'oxydation le mélange formant les esters d'acides 2,5-diarylamino-3,6-dihydrotéréphtaliques tel qu'on l'obtient par réaction d'un ester succinylosuccinique avec une arylamine.

4. Procédé suivant une ou plusieurs des revendications 1 à 3, caractérisé en ce qu'on utilise un composé d'ammonium quaternaire de formules

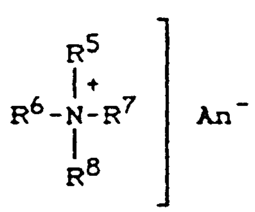

(III)

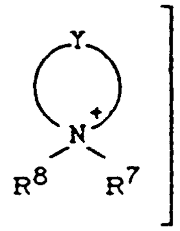 $An^-$ (IV)

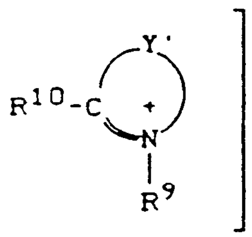 $An^-$ (V)

dans lesquelles

$R^5$, $R^6$, $R^7$, $R^8$ et $R^9$ sont des restes d'hydrocarbures en $C_1$ à $C_{54}$ dont la chaîne carbonée peut être interrompue par 1 à 15 atomes d'oxygène,

$R^{10}$ est l'hydrogène ou un substituant,

Y, Y′ sont les termes restants d'un noyau de préférence pentagonal ou hexagonal ou d'un système de noyaux et

$An^-$ désigne un reste anionique.

5. Procédé suivant l'une ou plusieurs des revendications 1 à 4, caractérisé en ce qu'on utilise, par rapport à l'ester d'acide 2,5-diarylamino-3,6-dihydrotéréphtalique, 0,1 à 15 % en poids, de préférence 0,5 à 8 % en poids de composé d'ammonium quaternaire.

6. Procédé suivant une ou plusieurs des revendications 1 à 5, caractérisé en ce qu'on utilise l'acide anthraquinone-2-sulfonique comme agent de transfert d'oxygène.

7. Procédé suivant une ou plusieurs des revendications 1 à 6, caractérisé en ce qu'on utilise comme alcool, le méthanol, l'éthanol ou l'isopropanol.

8. Procédé suivant une ou plusieurs des revendications 1 à 7, caractérisé en ce qu'on effectue l'oxydation à une température de 70 à 130°C.

9. Procédé suivant une ou plusieurs des revendications 1 à 8, caractérisé en ce qu'on effectue l'oxydation au point d'ébullition du mélange réactionnel.